# EUROPEAN PATENT SPECIFICATION

(11) **EP 3 568 071 B1**
(45) Date of publication and mention of the grant of the patent: **05.05.2021**
(21) Application number: 18701407.1
(22) Date of filing: 09.01.2018
(51) Int. Cl.: A61B 5/08, A61B 5/083, G01N 21/3504

(54) **CAPNOGRAPHY WITH DETECTOR AND INTEGRATED BANDPASS FILTER**
KAPNOGRAFIE MIT DETEKTOR UND INTEGRIERTEM BANDPASSFILTER
CAPNOGRAPHIE AVEC DÉTECTEUR ET FILTRE PASSE-BANDE INTÉGRÉ

(30) Priority: 16.01.2017 US 201762446622 P
(43) Date of publication of application: 20.11.2019
(73) Proprietor: Koninklijke Philips N.V., 5656 AG Eindhoven (NL)
(72) Inventor: JIANG, Zhi-Xing, 5656 AE Eindhoven (NL); JANDO, Szilveszter Cseh, 5656 AE Eindhoven (NL)
(74) Representative: Roche, Denis
(86) International application number: PCT/EP2018/050386
(87) International publication number: WO 2018/130497

(56) References cited:
- EP-A1- 1 876 652
- RU-C1- 2 215 473
- US-A- 4 859 858
- US-A- 5 800 360
- RODRIGO MARIA T ET AL.: "Process technology to integrate polycrystalline uncooled PbSe infrared detectors on interference filters", PROCEEDINGS OF SPIE, vol. 5251, 19 February 2004 (2004-02-19), pages 97-102, XP040252852, DOI: 10.1117/12.512601

## Description

### FIELD

The following relates generally to the capnography arts, medical monitoring arts, infrared detector arts, and related arts.

### BACKGROUND

A capnometer according to the state of the art is for instance described in US4859858. A capnometer measures the concentration or partial pressure of carbon dioxide (CO₂) in respiratory gases. The CO₂ waveform can provide information such as end-tidal carbon dioxide (etCO₂) which is a useful vital sign in assessing patients with respiratory problems, for assessing efficacy of mechanical respiration, and so forth.

In a typical capnometer design, a respired gas flow is accessed in either a mainstream configuration in which a CO₂ measurement cell is in-line with the respiratory circuit (e.g. in the mechanical ventilator airflow circuit) or in a sidestream configuration in which respired gas is drawn off the main flow using a pump. Infrared light is transmitted through respired gas flow. CO₂ absorbs significantly in the infrared, with an absorption peak at about 4.26 micron. In a typical optical detection setup, a bandpass filter having a pass band in the 3-5.5 micron range is used to isolate the CO₂-sensitive infrared spectral range, and a lead selenide (PbSe) detector made of a thin film of lead selenide deposited on a quartz substrate is used to detect the transmitted infrared light intensity. The PbSe film exhibits photoconductivity for infrared light in the wavelength range of 3 to 5.5 microns. As both the photoconductivity of the PbSe layer and the central wavelength of the pass band of the bandpass filter can vary with temperature, temperatures of both the PbSe detector and the bandpass filter typically are accurately monitored by thermocouples or other temperature sensors. These sensors provide feedback control for maintaining PbSe detector and the filter at the designed operating temperature or compensation of the ambient temperature change through special calibration and algorithm.

The following discloses new and improved systems that address the above referenced issues, and others.

### SUMMARY

The invention is defined in the appended claims.

One advantage resides in providing a more compact infrared light detector.

Another advantage resides in providing a more compact capnometer.

Another advantage resides in providing a capnometer with reduced components (i.e. fewer parts).

Another advantage resides in providing an infrared light detector with reduced reflection loss.

Another advantage resides in providing a capnometer with improved sensitivity.

Another advantage resides in providing a more accurate, consistent and correlated measurement of temperatures of both IR sensing element (PbSe) and IR selection element (narrow bandpass filter) with improved temperature control and/or compensation of ambient temperature change.

### BRIEF DESCRIPTION OF THE DRAWINGS

The drawings are only for purposes of illustration and are not to be construed as limiting the invention.
FIGURE 1 diagrammatically illustrates a capnometer.
FIGURE 2 diagrammatically illustrates a side sectional view of the integrated PbSe detector and bandpass filter component of the capnometer of FIGURE 1.
FIGURES 3 and 4 diagrammatically illustrate side sectional views of alternative integrated PbSe detector and bandpass filter components of the capnometer of FIGURE 1.

### DETAILED DESCRIPTION

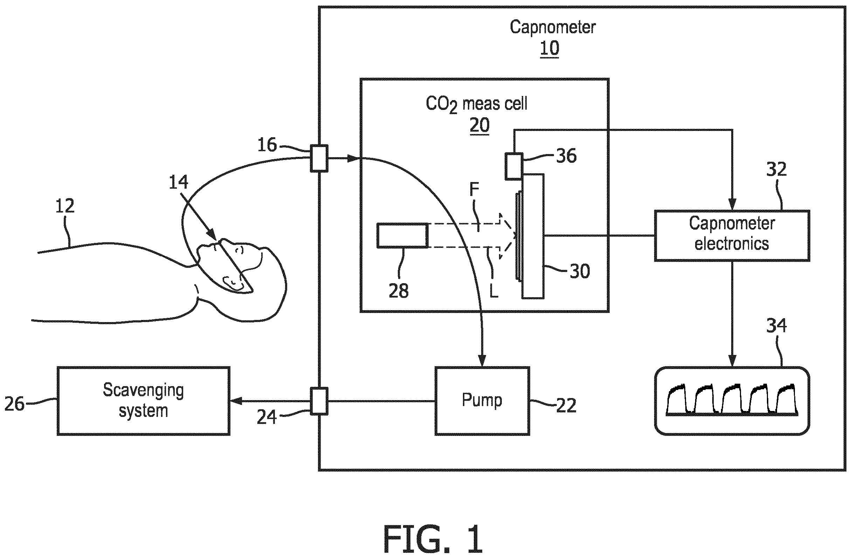

With reference to FIGURE 1, an illustrative capnometer **10** is connected with a patient **12** by a suitable patient accessory, such as a nasal cannula **14** in the illustrative example, or by an airway adaptor connecting with an endotracheal tube used for mechanical ventilation, or so forth. The patient accessory **14** may optionally include one or more ancillary components, such as an air filter, water trap, or the like (not shown). In the illustrative capnometer **10,** respired air is drawn from the patient accessory **14** into a capnograph air inlet **16** and through a carbon dioxide (CO₂) measurement cell **20** by an air pump **22.** The air is then discharged via an air outlet **24** of the capnometer **10** to atmosphere or, as in the illustrative embodiment, is discharged through the air outlet **24** into a scavenging system **26** to remove an inhaled anesthetic or other inhaled medicinal agent before discharge into the atmosphere.

The illustrative capnometer setup has a sidestream configuration in which respired air is drawn into the capnometer **10** using the pump **22,** and the CO₂ measurement cell **20** is located inside the capnometer **10.** The sidestream configuration is suitably used for a spontaneously breathing patient, i.e. a patient who is breathing on his or her own without assistance of a mechanical ventilator. In an alternative configuration, known as a mainstream configuration (not illustrated), the CO₂ measurement cell is located externally from the capnometer device housing, typically as a CO₂ measurement cell patient accessory that is inserted into the "mainstream" airway flow of the patient. Such a mainstream configuration may, for example, be employed in conjunction with a mechanically ventilated patient in which the CO₂ measurement cell patient accessory is designed to mate into an accessory receptacle of the ventilator unit, or is installed on an airway hose feeding into the ventilator.

The CO₂ measurement cell **20** comprises an infrared optical absorption cell in which carbon dioxide in the respired air drawn from the patient accessory **14** produces absorption in the infrared that is detected optically. CO₂ has an absorption peak at about 4.26 micron, and in some embodiments measurements are done within the 3-5.5 micron range inclusive or some sub-range of that range (preferably including 4.26 micron). To this end, a light source **28** emits light **L** over a spectrum that encompasses the desired measurement band (e.g. 3-5.5 micron or some sub-range thereof, preferably including 4.26 micron). The light **L** may extend spectrally beyond the detection spectral range, because it will be filtered as described below. In some embodiments the light source **28** may include an optical chopper, pulsed power supply, or the like in order to deliver the emitted light **L** as light pulses. The emitted light **L** transmits through a flow path **F** along which the respired gas flows. The flow path **F** may be defined by a tube or other conduit defining a cuvette with walls made of a plastic, glass, sapphire, or other material that is transparent over the range of interest (e.g. over 3-5.5 micron). In the sidestream arrangement, the pump **22** actively drives the flow of respired gas through the flow path **F;** in a mainstream configuration the flow may be driven by mechanical ventilation of the patient, and/or by active breathing of the patient.

An integrated device **30** including both a light detector layer and a bandpass filter operates to both filter light **L** so as to pass light in a pass band, e.g. 3-5.5 micron or some sub-range thereof (preferably including 4.26 micron) and to detect the light that passes the bandpass filter, e.g. using a lead selenide (PbSe) layer. Capnometer electronics **32** provide electrical biasing of the detector layer of the integrated device **30** and measure a detector signal (e.g. a voltage, current, or resistance) from the detector layer. For example, the electronics **32** may drive a fixed electric current through the PbSe layer and measure the voltage so as to output a voltage signal (or, alternatively, a resistance signal computed as V/I where V is the measured voltage and I is the applied current). Alternatively, the electronics **32** may apply a fixed voltage over the PbSe layer and measure the current so as to output a current signal (or, alternatively, a resistance signal computed as V/I where V is the applied voltage and I is the measured current). The applied and measured signals may in general be d.c. or a.c. or some combination (e.g. an a.c. signal superimposed on a d.c. bias). The electronics **32** also optionally include analog signal processing circuitry and/or digital signal processing (DSP) suitable for converting the detected signal into a capnography signal, e.g. a concentration or partial pressure of CO₂ in the respired gas flow, and optionally for performing further processing such as detecting a breath interval and/or an end-tidal CO₂ level (etCO₂ level). The conversion to CO₂ level can employ suitable empirical calibration - in general, higher CO₂ concentration or partial pressure in the flow **F** produces greater absorption and a reduced capnography signal voltage. The empirical calibration may take into account other factors such as flow rate or pressure, and/or the effects of other gases such as oxygen and nitrous oxide which can affect the infrared absorption characteristics, as is known in the art, and can be suitably programmed as a look-up table, mathematical equation, non-linear op-amp circuit, or so forth. In the case of the capnometer electronics **32** being implemented at least in part by DSP, such DSP may be implemented by a microcontroller or microprocessor or the like programmed by instructions stored on a read only memory (ROM), electronically programmable read-only memory (EPROM), CMOS memory, flash memory, or other electronic, magnetic, optical or other non-transitory storage medium that is readable and executable by the microcontroller or microprocessor or the like to perform the digital signal processing. For DSP processing, a front-end analog-to-digital (A/D) conversion circuit is typically provided to digitize the detector signal. An output component **34** is provided to output the capnometer signal or digital data generated by the capnometer electronics **32.** The illustrative output component is a display **34,** e.g. an LCD display or the like. The illustrative display **34** plots CO₂ concentration or partial pressure versus time as a trendline. Additionally or alternatively, the display may show a numerical value, e.g. of the etCO₂. The output component may additionally or alternatively take other forms, such as being or including (possibly in addition to the display **34**) a USB port or other data port via which the capnometer data may be read out. Moreover, the capnometer electronics **32** may perform additional functions such as monitoring a thermocouple, temperature diode, or other temperature sensor **36** that measures the operating temperature of the integrated device **30.** This is useful because a large change in temperature of the integrated device **30** can produce an undesired shift in the pass band of the bandpass filter and/or in the detector sensitivity. In some embodiments, the integrated device **30** may be mounted on or in thermal contact with a Peltier device or other thermoelectric cooling device (not shown) and the electronics **32** operates the thermoelectric cooling device in a feedback control mode using the temperature from the temperature sensor **36** to maintain the integrated device **30** at a design-basis operating temperature. It will be further appreciated that the capnometer **10** may include numerous other components not illustrated in simplified diagrammatic FIGURE 1, such as a pressure gauge and/or flow meter for monitoring the respired gas flow, a keypad or other user input components, and/or so forth.

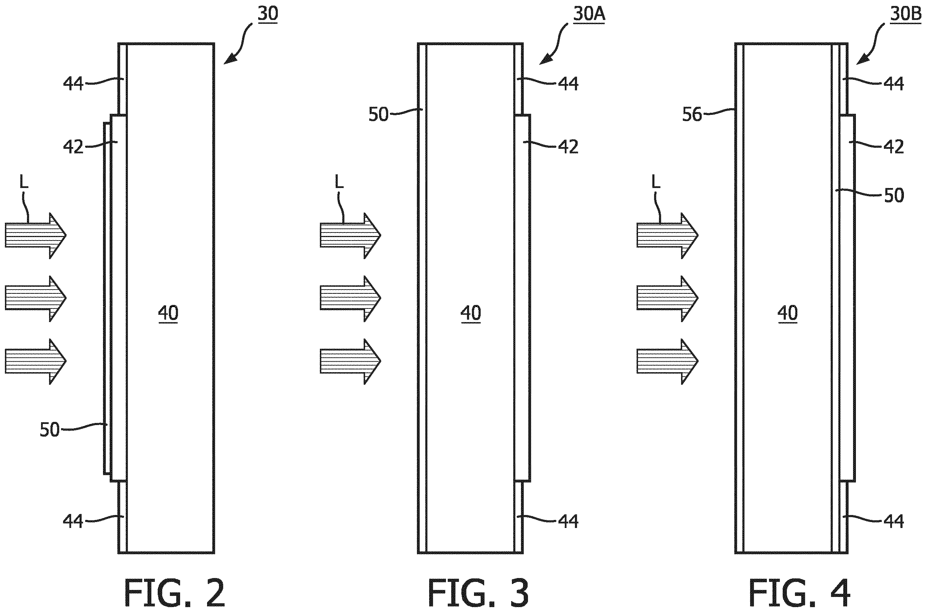

With reference now to FIGURE 2, a first embodiment of the integrated device **30** not according to the invention is shown. In this embodiment, the integrated device includes a substrate **40,** such as a quartz substrate by way of non-limiting illustrative example. The substrate **40** is preferably planar, e.g. a wafer or relatively thin chip having two flat opposing principal sides. A lead selenide (PbSe) layer **42** is disposed on one side of the substrate **40.** The PbSe layer **42** is sufficiently thick to absorb most light in the target range, e.g. 3-5.5 micron in some embodiments, or a smaller spectral range encompassing 4.26 micron in other embodiments. In some illustrative embodiments the PbSe layer **42** has a thickness between 0.5 micron and 2.5 micron inclusive. The PbSe layer **42** may, for example, be deposited by chemical bath deposition (CBD) or vapor phase deposition (VPD). Typically, CBD is preferable due to faster readily achievable deposition rates. The deposited PbSe layer **42** is usually a polycrystalline film. Electrodes **44** are also disposed on the substrate **40** and are electrically connected with the PbSe layer **42** to enable measurement of a photoconductivity signal of the PbSe layer **42,** e.g. a photoconductivity signal consisting of one of resistance, current, and voltage across the PbSe layer **42.** The electrodes **44** may, for example, be gold or silver pads disposed over ends of the PbSe layer **42** or disposed directly on the substrate **40** adjacent the ends of the PbSe layer **42.** The electrodes **44** may optionally include an adhesion layer and/or a diffusion barrier layer for promoting adhesion of the gold or silver pad layer to the PbSe and/or substrate material. The adhesion and/or diffusion barrier layer(s) may, for example, comprise one or more layers of chromium, titanium, titanium-tungsten, nickel, or so forth.

With continuing reference to FIGURE 2, the integrated device **30** further includes a bandpass filter layer **50,** which in the embodiment of FIGURE 2 is disposed on top of the PbSe layer **42.** As shown in FIGURE 2, the light **L** generated by the light source **28** (see FIGURE 1) passes through the bandpass filter layer **50** to reach the PbSe layer **42;** thus, photoconductivity induced in the PbSe layer **42** by the light **L** is limited to the spectral portion of light **L** lying in the pass band of the bandpass filter layer **50.** The bandpass filter layer **50** may be a broadband filter comprising a single layer or a plurality of layers operating by bulk absorption and/or optical interference to define a relatively broad pass band, e.g. 3-5.5 micron passband in some embodiments. Alternatively, the bandpass filter layer **50** may be a narrowband filter having a narrow pass band. In this approach the bandpass filter layer **50** comprises a multi-layer stack defining an interference filter. Such an interference filter can be designed using ray tracing approaches or other optical filter design techniques to have a narrow pass band tailored transitions between the pass band and the surrounding "stop" bands. Thus, for example, it is contemplated to design the bandpass filter layer **50** as a multi-layer stack defining an interference filter with a narrow pass band that encompasses a narrow transmission band at wavelength of 4.26 micron (the principal absorption line of CO₂) but does not encompass the absorption lines of other gases such as oxygen or nitrous oxide. Such an approach can simplify design of the signal processing circuitry (e.g. DSP or analog signal processing circuitry) of the electronics **32** (see FIGURE 1) by reducing or eliminating the correction for these interfering gases when converting the photoconductivity signal measured for the PbSe layer **42** into a CO₂ concentration or partial pressure.

Disposing the bandpass filter layer **50** on top of the PbSe layer **42** provides an additional benefit, namely refractive index matching. PbSe has a refractive index of about n=4.9 for the wavelength of 4.26 micron. When the infrared light impinges on a bare PbSe surface, transmission of the infrared light into the PbSe layer is reduced significantly by reflection at the large refractive index step from air (n=1.0) to PbSe (n=4.9). By some estimates, 44% of the light is reflected at a bare PbSe surface, leaving only 56% of the light to penetrate into the PbSe. In the integrated device **30** of FIGURE 2, by coating the PbSe layer **42** with the bandpass filter layer **50,** the refractive index step is reduced thus reducing the optical reflection loss as compared with a bare PbSe surface. To reduce reflection loss, the bandpass filter layer **50** should comprise material with refractive index that is intermediate between n=1.0 of air and n=4.9 of PbSe. Some suitable materials include, by way of non-limiting example: Aluminum Oxide (Al₂O₃), Titanium Oxide (TiO₂), Cryolite, Magnesium Fluoride (MgF₂), Calcium Fluoride (CaF₂), Zinc Selenide (ZnSe), Zirconia (ZrO₂), Zinc Sulfide (ZnS), Spinel (MgAl₂O₄), Lithium Fluoride (LiF), Lead Fluoride (PbF₂), Cadmium Fluoride (CdF₂), Cadmium Sulfide (CdS), Zinc Oxide (ZnO), Silicon, Silicon Dioxide (SiO₂), Germanium, Germanium Oxide (GeO₂), Lead Telluride, Gallium Arsenide (GaAs), Thorium Fluoride, Hafnium Oxide, Tantalum Oxide, Niobium Oxide, Cadmium Sulfide, Antimony Fluoride, Yttrium Fluoride, or various multilayer combinations thereof (e.g. to define an interference filter).

One potential difficulty with the integrated device **30** of FIGURE 2 is that the PbSe layer **42,** e.g. deposited by CBD or CVD, is typically a polycrystalline layer having relatively high surface roughness. When the bandpass filter layer **50** is deposited on top of the PbSe layer **42,** the resulting bandpass filter layer **50** may assume roughness or lateral variability that is comparable with the surface roughness of the underlying polycrystalline PbSe layer **42.** For some bandpass filter designs, such as narrowband interference filters, such roughness or lateral variability disrupts the "ideal" filter design in which perfectly planar layers of the multilayer stack cooperatively generate constructive and destructive interferences that define the optical pass band. One way to improve the situation is to employ mechanical or chemomechanical polishing of the surface of the PbSe layer **42** prior to deposition of the bandpass filter layer **50** in the embodiment of FIGURE 2.

With reference to FIGURES 3 and 4, alternative embodiment integrated devices **30A, 30B** are presented. In FIGURES 3 and 4, components corresponding to components of the integrated device **30** of FIGURE 2 (namely the substrate **40,** PbSe layer **42,** electrodes **44,** and bandpass filter layer **50**) are labeled with corresponding reference numbers. Either the integrated device **30A** of FIGURE 3, which is not in accordance with the invention, or the integrated device **30B** of FIGURE 4, which is in accordance with the invention, may be substituted for the integrated device **30** of FIGURE 1, with the light **L** from the light source **28** oriented as indicated in respective FIGURES 3 and 4. In particular, both the integrated devices **30A, 30B** have backside-illuminated configurations in which the light **L** impinges on the side of the substrate **40** opposite from the side on which the PbSe layer **42** is disposed. The alternative integrated device embodiments **30A, 30B** of respective FIGURES 3 and 4 are described in turn below.

With particular reference to FIGURE 3, to ensure the light **L** passes through the bandpass filter layer **50** to reach the PbSe layer **42,** in the configuration of FIGURE 3 the bandpass filter layer **50** is disposed on the side of the substrate **40** opposite the side on which the PbSe layer **42** is disposed. That is, the bandpass filter layer **50** is disposed on the side on which the light **L** first impinges. Said another way, the integrated device **30A** of FIGURE 3 differs from the integrated device **30** of FIGURE 2 in that the bandpass filter layer **50** is moved from being deposited on top of the PbSe layer **42** to being deposited on the side of the substrate **40** opposite the side on which the PbSe layer **42** is deposited. The arrangement of FIGURE 3 has a potential advantage over the arrangement of FIGURE 2 in that the bandpass filter layer **50** in FIGURE 3 is deposited onto the (backside) surface of the substrate **40,** which is expected to be relatively smooth, rather than being deposited onto the possibly rougher surface of the polycrystalline PbSe layer **42.** Thus, the integrated device embodiment **30A** may be preferable from a fabrication standpoint in the case of the bandpass filter layer **50** being designed as a multilayer stack with precise thin layer thicknesses defining a narrow pass band. The integrated device **30A** also presents an alternative solution to the problem of high reflection from the bare PbSe surface. In the embodiment **30A** of FIGURE 3, the light **L** passes through the substrate **40** into the PbSe layer **42,** so that the refractive index step encountered is between the refractive index of the substrate **40** and the refractive index of the PbSe layer **42.** So long as the substrate **40** has a refractive index that is intermediate between that of air (n=1.0) and PbSe (n=4.9) this configuration should reduce reflection loss. A further design constraint for the integrated device **30A** of FIGURE 3 is that the substrate **40** should be transparent for the pass band of the bandpass filter layer **50,** or in some embodiments at least should be transparent for infrared light at 4.26 micron. By way of some non-limiting examples, in some embodiments of the integrated device **30A** the substrate which is transparent for infrared light at 4.26 micron comprises silicon, germanium, zinc selenide, sapphire, titanium oxide, cryolite, magnesium fluoride, zirconia, zinc sulfide, lead fluoride, cadmium fluoride, cadmium sulfide, zinc oxide, tantalum oxide, niobium oxide, antimony fluoride or zerodur.

With particular reference to FIGURE 4, in the integrated device **30B** the bandpass filter layer **50** is on the same side of the substrate **40** as the PbSe layer **42** (same as in the embodiment of FIGURE 2), but in the integrated device **30B** of FIGURE 4 the bandpass filter **50** is disposed between the substrate **40** and the PbSe layer **42.** This arrangement again ensures that the (backside-impinging) light **L** passes through the bandpass filter layer **50** to reach the PbSe layer **42,** so that only the portion of light **L** that lies in the pass band of the bandpass filter layer **50** reaches the PbSe layer. As with the embodiment of FIGURE 3, the embodiment of FIGURE 4 has the potential advantage over the arrangement of FIGURE 2 that the bandpass filter layer **50** is deposited onto the (frontside) surface of the substrate **40,** which is expected to be relatively smooth, rather than being deposited onto the possibly rougher surface of the polycrystalline PbSe layer **42.** Thus again, the integrated device embodiment **30B** may be preferable from a fabrication standpoint in the case of the bandpass filter layer **50** being designed as a multilayer stack with precise thin layer thicknesses defining a narrow pass band. In the integrated device **30B,** the problem of high reflection from the bare PbSe surface is addressed by the light **L** encountering the smaller refractive index step between the bandpass filter layer **50** and the PbSe layer **42** and/or the ensuing destructive interference effect. So long as the material of the bandpass filter **50** has refractive index that is intermediate between that of air (n=1.0) and PbSe (n=4.9) this configuration should reduce reflection loss.

Although reflection loss at the interface to the PbSe layer **42** is addressed in the integrated device **30B** of FIGURE 4 as just described, there is potential for reflection loss at the "backside" of the substrate **40,** that is, at the side upon which the light **L** impinges. To reduce this reflection loss, an anti-reflective (AR) coating layer **56** is optionally disposed on the "backside" of the substrate **40,** i.e. on the side opposite from the side of the substrate **40** on which the PbSe **42** and bandpass filter layer **50** are disposed. The AR coating layer **56** is suitably any material with refractive index intermediate between that of air (n=1.0) and the refractive index of the substrate **40.** Alternatively, the AR coating layer **56** may be a multi-layer stack defining an interference filter having a pass band aligning (at least approximately) with that of the bandpass filter layer **50.**

As with the embodiment of FIGURE 3, a further design constraint for the integrated device **30B** of FIGURE 4 is that the substrate **40** should be transparent for the pass band of the bandpass filter layer **50,** or in some embodiments at least should be transparent for infrared light at 4.26 micron. By way of some non-limiting examples, in some embodiments of the integrated device **30B** the substrate which is transparent for infrared light at 4.26 micron comprises silicon, germanium, zinc selenide, sapphire, or zerodur.

In the embodiment **30B** of FIGURE 4, the PbSe layer **42** is deposited onto the bandpass filter layer **50.** This requires that the bandpass filter layer **50** be impervious to the wet chemical bath used in the chemical bath deposition (CBD) of the PbSe layer **42,** or alternatively requires that the bandpass filter layer **50** be impervious to the gas-phase chemistry if vapor phase deposition (VPD) is used to deposit the PbSe layer **42.** If the materials of the bandpass filter layer **50** are not capable of withstanding this wet or vapor phase chemistry, then it is contemplated to deposit a thin passivation layer on top of the bandpass filter layer **50** prior to CBD or VPD deposition of the PbSe layer **42.**

In the embodiment **30A** of FIGURE 3, the PbSe layer **42** may be deposited either before or after deposition of the bandpass filter layer **50.** If the PbSe layer **42** is deposited after the bandpass filter layer **50,** then the bandpass filter layer **50** should either be impervious to the CBD or VPD deposition chemistry, or the bandpass filter layer **50** should be protected by a permanent thin passivation layer or a temporary protective mask layer (e.g. a photoresist layer) that can be removed after deposition of the PbSe layer **42.**

In the illustrative embodiments, PbSe is used as the infrared light absorbing layer **42.** More generally, the PbSe layer is contemplated to be replaced by another infrared light absorbing layer such as a mercury cadmium telluride layer or an indium antimonide layer. That is, in any of the illustrative embodiments of the integrated component **30, 30A, 30B,** the illustrative PbSe layer **42** is contemplated to be replaced by a mercury cadmium telluride layer, an indium antimonide layer, or other infrared light absorbing layer that exhibits photoconductivity in response to illumination by infrared light in a spectral range encompassing the 4.26 micron absorption line for CO₂.

## Claims

1. A capnometer (10) comprising:
an integrated device (30B) comprising a substrate (40), an infrared light absorbing layer (42) disposed on the substrate, and a bandpass filter layer (50) disposed on the substrate;
a light source (28) arranged to emit light (L) that passes through the bandpass filter layer of the integrated device to reach the infrared light absorbing layer of the integrated device; and
electronics (32) connected with the infrared light absorbing layer of the integrated device to measure a photoconductivity signal of the infrared light absorbing layer, the electronics including signal processing circuitry to convert the photoconductivity signal to a carbon dioxide partial pressure or concentration value;
wherein the bandpass filter layer (50) and the infrared light absorbing layer (42) of the integrated device (30B) are disposed on a same side of the substrate (40) with the bandpass filter layer disposed between the substrate and the infrared light absorbing layer, and the substrate of the integrated device is transparent for the light (L) emitted by the light source (28) at least over the pass band of the bandpass filter layer of the integrated device;
wherein the capnometer (10) further comprises a carbon dioxide CO2 measurement cell (20) including a flow path (F) through which respired gas flows and
wherein the light source (28) is arranged to emit the light (L) to pass through the flow path (F) of the CO2 measurement cell before reaching the bandpass filter layer (50) of the integrated device (30B).

2. The capnometer (10) of claim 1 wherein the integrated device (30B) further comprises:
an anti-reflective (AR) coating layer (56) disposed on a side of the substrate (40) opposite from the side on which are disposed the infrared light absorbing layer (42) and the bandpass filter layer (50).

3. The capnometer (10) of any one of claims 1-2 wherein the pass band of the bandpass filter layer (50) of the integrated device (30B) encompasses 4.26 micron

4. The capnometer (10) of any one of claims 1-3 wherein the electronics (32) are connected with the infrared light absorbing layer (42) of the integrated device (30B) to measure the photoconductivity signal of the infrared light absorbing layer consisting of one of resistance, current, and voltage across the infrared light absorbing layer.

5. The capnometer (10) of any one of claims 1-4 further comprising:
a temperature sensor (36) thermally connected with the integrated device (30B);
wherein the electronics (32) are connected with the temperature sensor to monitor the temperature of the integrated device.

6. The capnometer (10) of any one of claims 1-5 wherein the infrared light absorbing layer (42) of the integrated device (30B) is a lead selenide (PbSe) layer

## Patentansprüche

1. Ein Kapnometer (10), der Folgendes umfasst:
ein integriertes Gerät (30B), das ein Substrat (40), eine auf dem Substrat angeordnete Infrarotlicht absorbierende Schicht (42) und eine auf dem Substrat angeordnete Bandfilterschicht (50) umfasst;
eine Lichtquelle (28), die Licht (L) emittiert, das die Bandfilterschicht des integrierten Geräts durchdringt, um die Infrarotlicht absorbierende Schicht des integrierten Geräts zu erreichen; und
eine Elektronik (32), die mit der Infrarotlicht absorbierenden Schicht des integrierten Geräts verbunden ist, um ein Lichtleitfähigkeitssignal der Infrarotlicht absorbierenden Schicht zu messen, wobei die Elektronik eine Signalverarbeitungsschaltung umfasst, um das Lichtleitfähigkeitssignal in einen Kohlendioxid-Partialdruck oder Konzentrationswert umzuwandeln;
wobei die Bandfilterschicht (50) und die Infrarotlicht absorbierende Schicht (42) des integrierten Geräts (30B) auf der gleichen Seite des Substrats (40) angeordnet sind, und wobei sich die Bandfilterschicht zwischen dem Substrat und der Infrarotlicht absorbierenden Schicht befindet, und wobei das Substrat des integrierten Geräts für das von der Lichtquelle (28) emittierte Licht (L) zumindest über das Band der Bandfilterschicht des integrierten Geräts transparent ist;
wobei das Kapnometer (10) zudem eine Kohlendioxid-C02-Messzelle (20) umfasst, die einen Strömungsweg (F) aufweist, durch den Atemluft strömt, und
wobei die Lichtquelle (28) das Licht (L) so emittiert, dass es den Strömungsweg (F) der C02-Messzelle durchläuft, bevor es die Bandfilterschicht (50) des integrierten Geräts (30B) erreicht.

2. Das Kapnometer (10) gemäß Anspruch 1, wobei das integrierte Gerät (30B) zudem Folgendes umfasst:
eine Antireflexions-(AR)Schicht (56), die sich auf der Seite des Substrats (40) befindet, die der Seite gegenüberliegt, auf der die Infrarotlicht absorbierende Schicht (42) und die Bandfilterschicht (50) angeordnet sind.

3. Das Kapnometer (10) gemäß einem der Ansprüche 1 bis 2, wobei das Band der Bandfilterschicht (50) des integrierten Geräts (30B) 4,26 Mikrometer umfasst.

4. Das Kapnometer (10) gemäß einem der Ansprüche 1 bis 3, wobei die Elektronik (32) mit der Infrarotlicht absorbierenden Schicht (42) des integrierten Geräts (30B) verbunden ist, um das Lichtleitfähigkeitssignal der Infrarotlicht absorbierenden Schicht zu messen, das über der Infrarotlicht absorbierenden Schicht jeweils entweder aus Widerstand, Strom und Spannung besteht.

5. Das Kapnometer (10) gemäß einem der Ansprüche 1 bis 4, das zudem Folgendes umfasst:
einen Temperatursensor (36), der thermisch mit dem integrierten Gerät (30B) verbunden ist;
wobei die Elektronik (32) mit dem Temperatursensor verbunden ist, um die Temperatur des integrierten Geräts zu überwachen.

6. Das Kapnometer (10) gemäß einem der Ansprüche 1 bis 5, wobei es sich bei der Infrarotlicht absorbierenden Schicht (42) des integrierten Geräts (30B) um eine Bleiselenid-(PbSe)Schicht handelt.

## Revendications

1. Capnomètre (10) comprenant :
un dispositif (30B) intégré comprenant un substrat (40), une couche absorbant la lumière infrarouge (42) étant disposée sur le substrat, et une couche de filtre passe-bande (50) étant disposée sur le substrat ;
une source lumineuse (28) conçue pour émettre la lumière (L), laquelle passe à travers la couche de filtre passe-bande du dispositif intégré pour atteindre la couche absorbant la lumière infrarouge du dispositif intégré ; et
une électronique (32) connectée à la couche absorbant la lumière infrarouge du dispositif intégré pour mesurer un signal de photoconductivité de la couche absorbant la lumière infrarouge, l'électronique comprenant un circuit de traitement du signal pour convertir le signal de photoconductivité en une pression partielle ou une valeur de concentration de dioxyde de carbone,
dans lequel la couche de filtre passe-bande (50) et la couche absorbant la lumière infrarouge (42) du dispositif (30B) intégré sont disposées sur un même côté du substrat (40), la couche de filtre passe-bande étant disposée entre le substrat et la couche absorbant la lumière infrarouge, et le substrat du dispositif intégré est transparent pour la lumière (L) émise par la source lumineuse (28) au moins sur la bande passante de la couche de filtre passe-bande du dispositif intégré ;
dans lequel ledit capnomètre (10) comprend en outre une cellule de mesure de dioxyde de carbone CO2 (20) comprenant un trajet d'écoulement (F) à travers lequel s'écoule le gaz respiré, et dans lequel la source lumineuse (28) est conçue pour émettre la lumière (L) pour traverser le trajet d'écoulement (F) de la cellule de mesure de CO2 avant d'atteindre la couche de filtre passe-bande (50) du dispositif (30B) intégré.

2. Capnomètre (10) selon la revendication 1, dans lequel le dispositif (30B) intégré comprend en outre :
une couche de revêtement antireflet (AR) (56) disposée sur un côté du substrat (40) opposé au côté sur lequel la couche absorbant la lumière infrarouge (42) et la couche de filtre passe-bande (50) sont disposées.

3. Capnomètre (10) selon l'une quelconque des revendications 1-2, dans lequel la bande passante de la couche de filtre passe-bande (50) du dispositif intégré (30B) englobe 4,26 microns.

4. Capnomètre (10) selon l'une quelconque des revendications 1 à 3, dans lequel l'électronique (32) est connectée à la couche absorbant la lumière infrarouge (42) du dispositif intégré (30B) pour mesurer le signal de photoconductivité de la couche absorbant la lumière infrarouge consistant en une résistance, un courant et une tension au niveau de la couche absorbant la lumière infrarouge.

5. Capnomètre (10) selon l'une quelconque des revendications 1 à 4, comprenant en outre :
un capteur de température (36) relié thermiquement au dispositif intégré (30B), dans lequel l'électronique (32) est connectée au capteur de température pour surveiller la température du dispositif intégré.

6. Capnomètre (10) selon l'une quelconque des revendications 1 à 5, dans lequel la couche absorbant la lumière infrarouge (42) du dispositif (30B) intégré est une couche de séléniure de plomb (PbSe).
